# EUROPEAN PATENT SPECIFICATION

(11) **EP 2 246 120 B1**
(45) Date of publication and mention of the grant of the patent: **23.10.2013**
(21) Application number: 09715772.1
(22) Date of filing: 17.02.2009
(51) Int. Cl.: B05B 5/057, A61L 9/14, B05B 5/025

(54) **ELECTROSTATIC ATOMIZER**
ELEKTROSTATISCHER ZERSTÄUBER
PULVÉRISATEUR ÉLECTROSTATIQUE

(30) Priority: 26.02.2008 JP 2008044849
(43) Date of publication of application: 03.11.2010
(73) Proprietor: Panasonic Corporation, Osaka 571-8501 (JP)
(72) Inventor: NAKADA, Takayuki, Osaka 540-6207 (JP); MIYATA, Takahiro, Osaka 540-6207 (JP); SUDA, Hiroshi, Osaka 540-6207 (JP); ASANO, Yukiyasu, Osaka 540-6207 (JP); MACHI, Masaharu, Osaka 540-6207 (JP)
(74) Representative: Appelt, Christian W.
(86) International application number: PCT/JP2009/052668
(87) International publication number: WO 2009/107513

(56) References cited:
- EP-A1- 1 666 156
- JP-A- 2004 285 036
- JP-A- 2004 285 036
- JP-A- 2005 232 015
- JP-A- 2006 095 502
- JP-A- 2006 095 502
- JP-A- 2007 167 796
- JP-A- 2007 167 796

## Description

### Technical Field

The present invention is directed to an electrostatic atomizing device which generates a mist of charged minute water particles by causing an electrostatic atomizing phenomenon.

### Background Art

In the past, as disclosed in Japanese laid-open patent publication No. 2007-167796, there is known an electrostatic atomizing device configured to atomize water (e.g. tap water) to generate a mist of charged minute water particles of nanometer size.

The aforementioned mist of charged minute water particles includes a radical such as a hydroxy radical. Such radical possesses an oxidation action. Therefore, the electrostatic atomizing device is capable of producing such as a deodorization effect, a virus and/or mold filtration effect, and a virus and/or mold suppression effect.

For example, the above Japanese laid-open patent publication discloses the charged minute water particles is attached to and is percolated into a food product to make a sterilization, a deodorizing, a degradation of harmful materials, or a moisture retention (in short, a food preservation).

Notably, objects (e.g. food products) can be deteriorated by not only action of a bacteria but also oxidization. In particular, to prevent a deterioration caused by oxidization is necessary for preserving freshness of food products for a long time.

However, the mist of charged minute water particles generated from water fails to suppress or prevent the deterioration caused by oxidization. That is, the conventional electrostatic atomizing device is incapable of preventing oxidization.

### Disclosure of Invention

In view of the above insufficiency, the present invention has been aimed to propose an electrostatic atomizing device capable of suppressing and preventing oxidization.

The electrostatic atomizing device in accordance with the present invention includes a discharge electrode and a potential applying means. The potential applying means is configured to apply an electrical potential to the discharge electrode to atomize a liquid supplied to the discharge electrode. The electrostatic atomizing device further includes a reduced water providing means configured to supply reduced water as the liquid to the discharge electrode.

According to this invention, the mist of charged minute water particles is produced from the reduced water. The mist of charged minute water particles produced from the reduced water has a reduction action. Therefore, it is possible to suppress and prevent oxidization of a targeted object (in particular, oxidization caused by an oxidized radical contained in the mist of charged minute water particles). Thus, the electrostatic atomizing device is capable of suppressing and preventing a deterioration of the targeted object.

In a preferred embodiment, the reduced water providing means includes a water storage tank configured to store the reduced water and a liquid transporter configured to transport the reduced water stored in the water storage tank to the discharge electrode.

According to this embodiment, it is possible to provide a simplified structure of supplying the reduced water to the discharge electrode.

In a preferred embodiment, the reduced water is defined to contain a material which has a reduction action and is dissolved or dispersed in the form of a minute particle in the reduced water. The discharge electrode is formed to have at least one part made of the material. The reduced water providing means is defined by a water providing means, the discharge electrode, and the potential applying means. The water providing means is configured to supply water to the discharge electrode. The potential applying means is configured to apply the electrical potential to the discharge electrode to dissolve the material from the discharge electrode in the water supplied by the water provision unit and held by the discharge electrode or to disperse in the form of the minute particle the material from the discharge electrode in the water supplied by the water provision unit and held by the discharge electrode.

According to this embodiment, the reduced water is generated by dissolving the material forming at least one part of the discharge electrode in the water supplied to the discharge electrode or by dispersing in the form of the minute particle the material forming at least one part of the discharge electrode in the water supplied to the discharge electrode. Therefore, the reduced water need not be prepared. Further, it is possible to provide a simplified structure of supplying the reduced water to the discharge electrode.

In addition, the material is preferred to be platinum.

### Brief Description of Drawings

FIG. 1 is a schematic view illustrating an electrostatic atomizing device in accordance with a first embodiment, and
FIG. 2 is a schematic view illustrating an electrostatic atomizing device in accordance with a second embodiment.

### Best Mode for Carrying Out the Invention

### (first embodiment)

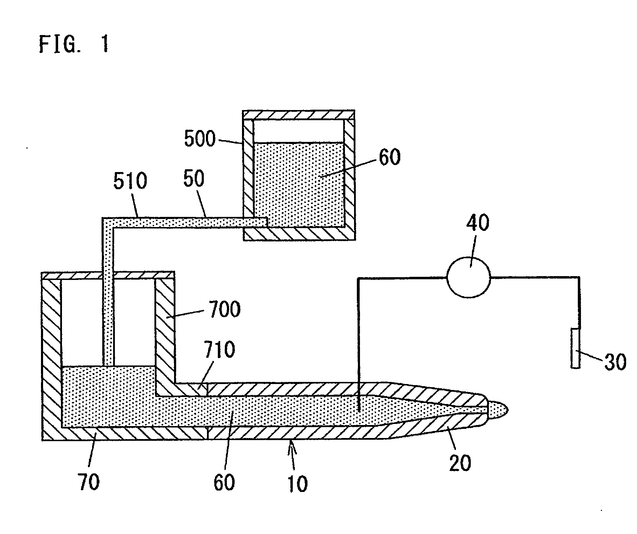

FIG. 1 shows an electrostatic atomizing device **10** which includes a discharge electrode (atomizing electrode) **20,** an opposed electrode **30,** and a voltage application device (voltage applying means) **40.** The electrostatic atomizing device **10** further includes a reduced water provision device (reduced water providing means) **50** configured to have the discharge electrode **20** hold reduce water **60.**

The discharge electrode **20** is made of metals and is shaped into a cylindrical shape (e.g. a circular cylindrical shape). The discharge electrode **20** has its front end portion (right end portion, in FIG. 1) which has its inner diameter made smaller towards its front end than at its rear end. The inner diameter at the front end of the discharge electrode **20** is selected such that the reduced water **60** outside the front end of the discharge electrode **20** can be kept in a spherical shape by its surface tension. In short, the reduced water **60** is prevented from flowing out from the front end of the discharge electrode **20.** Therefore, the reduced water **60** is held at the front end of the discharge electrode **20**.

In the electrostatic atomizing device **10** of the present embodiment, the discharge electrode **20** is attached to a reservoir **70.** The reservoir **70** includes a reservoir body **700,** for example. The reservoir body **700** is provided with a supply conduit **710** on a lower portion of its side surface. The supply conduit **710** has its inside communicate to an inside of the reservoir body **700,** and the reduced water **60** in the reservoir body **700** flows outside through the supply conduit **710**. The discharge electrode **20** is attached at an apex of the supply conduit **710.** Therefore, the reduced water **60** in the reservoir body **710** is supplied to an inside of the discharge electrode **20** through the supply conduit **710.**

The reduced water provision device **50** includes a tank **500** and a cylindrical-shaped liquid transporter **510**, and the tank **500** is a water storage tank configured to store the reduced water **60.** The liquid transporter **510** is configured to connect the tank **500** to the reservoir body **700.** The liquid transporter **510** acts as water distributing pipe for distributing the reduced water. Namely, the reduced water **60** stored in the tank **500** is transported to the reservoir body **700** via the liquid transporter **510.** The reduced water **60** in the reservoir body **700** flows into the discharge electrode **20** via the supply conduit **710**. As apparent from the above, the liquid transporter **510** is configured to transport the reduced water **60** stored in the tank **500** to the discharge electrode **20.** The illustrated instance shows the reduced water provision device **50** which utilizes gravity to supply the reduced water **60** to the discharge electrode **20.** However, the reduced water provision device **50** may be configured to utilize such as capillarity and a pump to supply the reduced water **60** to the discharge electrode **20.**

The voltage application device **40** is configured to apply a voltage between the discharge electrode **20** and the opposed electrode **30.** For example, the voltage application device **40** is a high voltage application device (high voltage applying means) configured to apply a voltage (e.g. voltage of 5000V) enough to atomize the reduced water **60** between the discharge electrode **20** and the opposed electrode **30** by use of an electrical power received from a commercial AC power source. The voltage application device **40** can be made by use of well known techniques and no detailed explanation is deemed necessary. It is noted that the voltage application device **40** of the present embodiment is configured to apply a negative potential to the discharge electrode **20** and to apply a ground potential (0V) to the opposed electrode **30.** In the present embodiment, the voltage application device **40** functions as a potential applying means configured to apply an electrical potential to the discharge electrode **20** to atomize the liquid supplied to the discharge electrode **20.** The voltage application device **40** need not apply the ground potential to the opposed electrode **30.** The opposed electrode **30** may be grounded instead of being supplied with the ground potential from the voltage application device **40.** In this case, the voltage application device **40** is configured to apply an electrical potential to the discharge electrode **20** only. The opposed electrode **30** can be replaced by a part of a housing (not shown) configured to house the electrostatic atomizing device **10** or a part of a main body (not shown) of an instrument to which the electrostatic atomizing device **10** is attached. In short, if the part of the housing or the main body is grounded, the electrostatic atomizing device **10** is not required to involve the opposed electrode **30.** Thus, it is possible to simplify a configuration of the electrostatic atomizing device **10.**

The opposed electrode **30** is opposed to the front end of the discharge electrode **20** and is spaced from the discharge electrode **20** by a predetermined distance. A distance between the discharge electrode **20** and the opposed electrode **30** is appropriately selected according to a usage pattern of the electrostatic atomizing device **10.** Further, the opposed electrode **30** is not required to be opposed to the discharge electrode in a strict sense. In brief, the opposed electrode **30** is allowed to be arranged such that the atomization of the reduced water **60** held by the discharge electrode **20** is successfully made by applying the voltage between the discharge electrode **20** and the opposed electrode **30**.

In the electrostatic atomizing device **10** of the present embodiment, for the purpose of generating the mist of charged minute water particles, the voltage application device **40** applies the voltage between the discharge electrode **20** and the opposed electrode **30** while the discharge electrode **20** has the reduced water **60** at its front end. Thereby, a Coulomb force acts on the reduced water **60** because an electric field is impressed to the reduced water **60** at the front end of the discharge electrode **20.** The Coulomb force causes the surface of the reduced water **60** to bulge locally, thereby forming a Taylor cone. Then, electric charges become concentrated at a tip of the Taylor cone to increase the electric field intensity and therefore the Coulomb force, thereby further developing the Taylor cone. Energy (repulsion caused by high-density charges) which acts on the reduced water **60** at the tip of the Taylor cone becomes high as charge density at the tip of the Taylor cone becomes high. Accordingly, upon the charge density at the tip of the Taylor cone exceeding a predetermined value, the Coulomb force exceeds the surface tension of the reduced water **60.** Thereby, the reduced water **60** is caused to disintegrate repeatedly (Rayleigh disintegration) to generate a large amount of the mist of charged minute water particles of nanometer sizes. The mist of charged minute water is charged positively or negatively.

Water in which a material (hereinafter called "reducing material", in the first and second embodiment) having a reduction action is dispersed in the form of a solid, minute particle can be adopted as the reduced water **60.** In addition, water in which the reducing material is dissolved can be adopted as the reduced water **60.** Herein, the reducing material is not limited to a material generally referred to as a reductant having reducing ability by itself. For example, the reducing material can be broadly defined as a material (e.g. platinum nano-size particle) which functions as a catalyst to quench active oxygen.

In the present embodiment, water in which a platinum minute particle is dispersed is adopted as the reduced water **60.** A platinum nano-size particle (e.g. a platinum minute particle of 2nm size) is adopted as the platinum minute particle. To electrostatically atomize this reduced water **60** generates the charged minute water particles of nanometer size which includes the platinum nano-size particle. Preferably, the charged minute particles include those particles of nanometer size which are distributed to show a peak in a particle size range of 15 to 30 nm.

Such the charged minute water particle is of nanometer size and is extremely small. Therefore, the charged minute water particles are delivered far and wide. After the charged minute water particles come into contact with a surface of a targeted object (e.g. a food, a skin, and a hair), the charged minute water particles percolate into the target object. Therefore, the charged minute water particle can deliver the platinum nano-size particle to an inside of the targeted object.

Although platinum is not a reductant in a strict sense, it is well known that the platinum nano-size particle produces an effect of catalytically quenching the active oxygen (that is, the platinum nano-size particle acts as a catalyst to bring about the reduction action). Therefore, on the surface or in the inside of the targeted object, the platinum nano-size particle functions as the catalyst for reduction. Accordingly, when the water in which the platinum nano-size particles are dispersed is adopted as the reduced water **60,** it is possible to prevent the targeted object from being oxidized. In particular, in the case of the targeted object being a food, it is possible to prevent not only a surface of the food but also an inside of the food from being oxidized. Thus, the freshness of the food can be preserved.

In the case of the targeted object being a skin and/or hair, the platinum nano-size particle suppresses the oxidization on a surface of the skin and/or hair and/or in an inside of the skin and/or hair. That is, the platinum nano-size particle can prevent the skin and hair from being deteriorated (aged). Further, the charged minute water particle of nanometer size containing the platinum nano-size particle goes into an inside of the body via the mouth or nose. In this case, it is possible to suppress the aging because the platinum nano-size particle suppresses the oxidization inside the body.

It is noted that the reduced water **60** is not limited to the water in which the platinum nano-size particles are dispersed. For example, water in which a material having the reduction action but platinum is dispersed can be adopted as the reduced water **60.** In addition, the reduced water **60** can be selected from such as hydrogen water (active hydrogen water) and ascorbic acid water. It is noted that the hydrogen water means water containing a large amount of hydrogen.

In the case of the hydrogen water being adopted as the reduced water **60,** the hydrogen water may be made by electrolysis of the water stored in the tank **500.** The hydrogen water produced in the tank **500** may be transported to the front end of the discharge electrode **20** by the liquid transporter **510** and subsequently may be electrostatically atomized.

Namely, the reduced water provision device **50** may include an electrolysis device (not shown) configured to produce the hydrogen water by electrolysis of the water stored in the tank **500**, in addition to the tank **500** and the liquid transporter **510.** According to this configuration, the reduced water **60** can be produced in the tank **500.** Therefore, it is not required to preliminarily generate the reduced water 60 by use of an external device. It is only required to supply water to the tank **500.**

To electrostatically atomizing the hydrogen water and the ascorbic acid water produces a mist of charged minute hydrogen water particles and a mist of charged minute ascorbic acid water particles, respectively. The hydrogen water and the ascorbic acid water are the reductant by themselves. Therefore, in the case of the reduced water **60** being selected from the hydrogen water and the ascorbic acid water, it is possible to suppress the deterioration caused by the oxidization because the reduced water **60** produces the reduction action on the surface or in the inside of the targeted object.

In the case of the discharge electrode **20** being supplied with water instead of the reduced water **60,** the voltage applied between the discharge electrode **20** and the opposed electrode **30** generates a free radical (e.g. [·H], [·OH], and [·O₂]) in the water at the front end of the discharge electrode **20.** In this case, an electron (e⁻) is provided to the Taylor cone of the water from the discharge electrode **20** when the voltage is applied to give a lower potential to the discharge electrode **20** than the opposed electrode **30.** Therefore, the electron combines with [·H] to produce H₂. As a result, [·OH] and [·O₂] remain in the water. Finally, the electrostatically atomization produces the charged minute water particle containing active oxygen. This negatively-charged minute water particle is defined to contain the active oxygen such as [·OH] and [·O₂].

By contrast, the electrostatic atomizing device **10** in accordance with the present embodiment generates the mist of the charged minute water particles containing the material having the reduction action. Further, the reducing material contained in the charged minute water particle has enough reduction ability to supersede or prevail the oxidation caused by the oxidized radical of [·OH] and [·O₂] contained in the charged minute water particle. Therefore, the oxidized radical of [·OH] and [·O₂] is prevented from producing the oxidation action, and the reduction action caused by the reducing material suppresses the oxidation.

As described in the above, the electrostatic atomizing device **10** of the present embodiment includes the discharge electrode **20,** the opposed electrode **30,** and the voltage application device **40**. The voltage application device **40** is configured to apply a voltage between the discharge electrode **20** and the opposed electrode **30** to atomize a liquid held by the discharge electrode **20.** The electrostatic atomizing device **10** further includes the reduced water provision device **50** configured to supply the reduced water **60** as the liquid to the discharge electrode **20.**

According to the electrostatic atomizing device **10,** the mist of charged minute water particles is produced from the reduced water **60.** Unlike charged minute water particle produced from mere water such as tap water, the charged minute water particle produced from the reduced water **60** has the reduction action. Therefore, it is possible to suppress and prevent oxidization of a targeted object (in particular, oxidization caused by an oxidized radical contained in the mist of charged minute water particles). Thus, the electrostatic atomizing device is capable of suppressing and preventing a deterioration of the targeted object.

In addition, the reduced water provision device **50** includes the tank **500** configured to store the reduced water **60** and the liquid transporter **510** configured to transport the reduced water **60** stored in the tank **50** to the discharge electrode **20.** Accordingly, it is possible to provide a simplified structure of supplying the reduced water **60** to the discharge electrode 20. In addition, it is possible to generate the charged minute water particle containing a minute particle having the reduction action irrespective of a material of the discharge electrode **20.**

Further, when the reduced water **60** is water having the reducing material dispersed therein, the reducing material floats in air in a state of being contained in the tiny charged minute water particle and comes into contact with the target. The charged minute water particle percolates into the targeted object because the charged minute water particle is tiny. Further, the charged minute water particle goes into the inside of the body via the mouth or nose. After the mist of the charged minute water particle is evaporated, the reducing material produces the reduction action on the surface or the inside of the targeted object and particularly suppresses the oxidization caused by the oxidized radical contained in the charged minute water particle. Accordingly, it is possible to suppress and prevent the oxidization of the targeted object. Thus, the deterioration of the oxidization of the targeted object can be suppressed and prevented. Further, in contrast to a situation where the reducing material alone flies and adhere to the target, the reduction due to the reducing material can be delayed by a time during which the water (charged minute water particles) is evaporated. Whereby, the reducing material can achieve the reduction only after it adheres and permeated into the target. Thus, the oxidization can be successfully suppressed and prevented by the reducing material.

### (second embodiment)

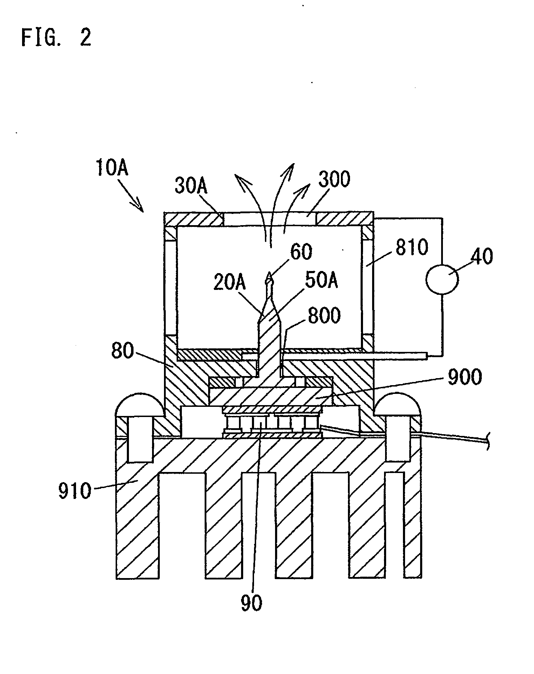

As shown in FIG. 2, the electrostatic atomizing device **10A** of the present embodiment includes the discharge electrode **20A,** the opposed electrode **30A,** the voltage application device **40,** a housing **80,** and a cooler **90.** Since the voltage application device **40** is the same as that of the first embodiment **1,** the voltage application device **40** is designated by the same reference number and no explanation is deemed necessary.

The discharge electrode **20A** is shaped into a column shape, for example. The discharge electrode **20A** has its outer diameter made smaller towards its front end than at its rear end. The discharge electrode **20A** is made of the reducing material. For example, the reducing material can be selected from platinum (Pt), zinc (Zn), silver (Ag), and titanium (Ti).

The housing **80** is shaped into a box shape which having its entire first surface (upper surface, in FIG. 2) opened. The housing **80** is provided in its second surface (lower surface, in FIG. 2) with a communication hole **800** communicating an inside of the housing **80** with an outside of the housing **80.** The discharge electrode **20A** has its front end inserted into the housing **80** via the communication hole **800.** The housing **80** is provided with window holes **810** in its side surface. The window **810** is provided to the housing **80** for introducing circumambient air into the housing **80.**

The opposed electrode **30A** is disposed on the first surface of the housing **80.** The opposed electrode **30A** is shaped into a plate shape having enough dimensions to cover an opening in the first surface of the housing **80.** Further, the opposed electrode **80** is provided with a spray hole **300** extending along its thickness direction. The spray hole **300** is designed for discharging the charged minute water particles produced in the housing **80** to the outside of the housing **80.**

The cooler **90** is adapted to cool the discharge electrode **20A.** The cooler **90** is a peltier unit **90** which includes a peltier module **900** and a heat dissipation fin **910** configured to contact with a heater region of the pletier module **900.** The cooler **90** is attached to the second surface of the housing **80** such that the peltier module **900** has its cooler region contacting with the rear end of the discharge electrode **20A.** The cooler **90** is driven to cool the discharge electrode such that the temperature of the discharge electrode **20A** becomes not greater than a dew point temperature of circumambient air. When the discharge electrode **20A** is cooled below the dew point temperature, moisture in the circumambient air is condensed on the surface of the discharge electrode **20A.** Therefore, dew is produced on the surface of the discharge electrode **20A.** That is, the cooler **90** is configured to supply water (dew condensation water) to the discharge electrode **20A** utilizing dew condensation (surface condensation). As apparent from the above, the cooler **90A** functions a water supplying means configured to supply water to the discharge electrode **20A.**

When the relatively high voltage (high voltage) is applied across the discharge electrode **20A** and the opposed electrode **30A,** the resulting energy given by the high voltage is likely to dissolve a part of the discharge electrode **20A** held in contact with the water or separate the same from the discharge electrode **20A** in the form of the minute particle. As a result, the water held by the discharge electrode **20A** is changed into the reduced water **60** containing the reducing material. In addition, corona discharge brings about separation of a part of the discharge electrode **20A** held in no contact with the water from the discharge electrode **20A.** This minute particle is dispersed into the water held by the discharge electrode **20A.** As a result, the water held by the discharge electrode **20A** is changed into the reduced water **60** containing the reducing material.

Namely, in the electrostatic atomizing device **10A,** a reduced water provision device (reduced water providing means) **50A** is defined by the discharge electrode **20A,** the voltage application device **40,** and the cooler **90.** The reduced water provision device **50A** is configured to apply a voltage between the discharge electrode **20A** and the opposed electrode **30A** (In other words, the electrical potential applied to the discharge electrode **20A)** to dissolve or disperse in the form of the minute particle the material of the discharge electrode **20A** in the water supplied to the discharge electrode **20A** by use of the cooler **90.**

In the electrostatic atomizing device **10A,** in order to generate the charged minute water particles, first the cooler **90** is driven to cool the discharge electrode **20A** to generate the dew on the surface of the discharge electrode **20A.** Subsequently, the voltage application device **40** is driven to apply the voltage between the discharge electrode **20A** and the opposed electrode **30A.** The Taylor cone is formed from the water held by the discharge electrode **20A** when a voltage (high voltage) not less than a predetermined voltage is applied between the discharge electrode **20A** and the opposed electrode **30A.** Further, the reducing material is dissolved in the Taylor cone. Alternatively, the reducing material is separated from the discharge electrode **20A** and is dispersed into the Taylor cone in the form of the minute particle. Therefore, the Taylor cone contains the reducing material.

Especially, the resulting energy given by the applied high voltage is likely to dissolve a part of the discharge electrode **20A** held in contact with the water or separate the same from the discharge electrode **20A** in the form of the minute particle. Therefore, the Taylor cone from the reduced water **60** containing the reducing water is produced.

In addition, corona discharge brings about separation of the reducing material composing a part of the discharge electrode **20A** held in no contact with the Taylor cone from the discharge electrode **20A.** This minute particle is dispersed into the Taylor cone when coming into contact with the Taylor cone. Therefore, the Taylor cone from the reduced water **60** containing the reducing water is produced.

Accordingly, the charged minute water particle containing the reducing material is produced by electrostatically atomizing this Taylor cone.

As described in the above, according to the electrostatic atomizing device **10A**, the reduced water **60** is generated by dissolving the material forming the discharge electrode **20A** in the water supplied to the discharge electrode **20A** or by dispersing in the form of the minute particle the material forming the discharge electrode **20A** in the water supplied to the discharge electrode **20A.** Therefore, the reduced water **60** need not be prepared. Further, it is possible to provide a simplified structure of supplying the reduced water **60** to the discharge electrode **20A**.

Herein, when the discharge electrode **20A** is made of platinum, the water in which the platinum nano-size particle is dispersed is produced as the reduced water **60**.

Although the present embodiment exemplifies an instance where the entire discharge electrode **20A** is made of the reducing material, the discharge electrode **20A** may have at least one part (especially, a surface part coming into contact with moisture) made of the reducing material. In the present embodiment, the water supplying means is the peltier unit **90** configured to cool the discharge electrode **20A.** However, the water supplying means may be a water provision device configured to supply water stored in a water tank to the front end of the discharge electrode **20A** by use of a transporting means. It is noted that the transporting means may be a device utilizing capillarity. In the present embodiment, the peltier unit **90** cools directly the discharge electrode **20A** to produce the dew on the surface of the discharge electrode **20A.** Alternatively, the dew may be produced on a surface of a cooling member (not shown) which is provided as separated parts from the discharge electrode **20A** by use of a cooling means such as the peltier unit **90.** In this case, the dew generated on the cooling member by dew condensation may be transported to the discharge electrode **20A** by the transporting means.

## Claims

1. An electrostatic atomizing device (10) comprising:
a discharge electrode (20);
a potential applying means (40) configured to apply an electrical potential to said discharge electrode (20) to atomize a liquid supplied to said discharge electrode (20); and
a reduced water providing means (50) configured to supply reduced water (60) as said liquid to said discharge electrode (20),
wherein the reduced water (60) is defined to contain a material which has a reduction action and is dissolved or dispersed in the form of a minute particle in the reduced water (60),
**characterized in that**
said discharge electrode (20) being formed to have at least one part made of said material,
said reduced water providing means (50) being defined by a water providing means,
said discharge electrode (20), and said potential applying means (40), said water providing means (50) being configured to supply water to said discharge electrode (20),
and
said potential applying means (40) being configured to apply the electrical potential to said discharge electrode (20) to dissolve or disperse in the form of the minute particle the material of said discharge electrode (20) in the water supplied by said water provision unit and supplied to said discharge electrode (20).

2. An electrostatic atomizing device (10) as set forth in claim 1, wherein
said material is platinum.

## Patentansprüche

1. Elektrostatische Zerstäubervorrichtung (10) mit:
einer Entladungselektrode (20);
Potentialanlegemittel (40), die ausgebildet sind zum Anlegen eines elektrischen Potentials an der Entladungselektrode (20), um eine Flüssigkeit, die an der Entladungselektrode (20) bereitgestellt ist, zu zerstäuben; und
Bereitstellungsmittel für reduziertes Wasser (50), die ausgebildet sind zum Bereitstellen von reduziertem Wasser (60) als die Flüssigkeit für die Entladungselektrode (20), wobei das reduzierte Wasser (60) dadurch definiert ist, dass es ein Material aufweist, welches eine Reduktionsreaktion auslöst und in Form von Partikeln in dem reduzierten Wasser (60) aufgelöst oder verteilt ist,
**dadurch gekennzeichnet, dass**
die Entladungselektrode (20) derart gebildet ist, dass sie einen Teil aufweist, der aus dem Material gebildet ist,
wobei die Bereitstellungsmittel für reduziertes Wasser (50) durch ein Wasserbereitstellungsmittel definiert ist, wobei die Entladungselektrode (20), die Potentialanlegemittel (40) und die Wasserbereitstellungsmittel (50) ausgebildet sind zum Bereitstellen von Wasser an der Entladungselektrode (20), und
die Potentialanlegemittel (40) ausgebildet sind zum Anlegen des elektrischen Potentials an der Entladungselektrode (20), um das Material der Entladungselektrode (20) in Form von kleinen Teilchen in dem Wasser, welches der Wasserbereitstellungseinheit und der Entladungselektrode (20) bereitgestellt wird, aufzulösen oder zu verteilen.

2. Elektrostatische Zerstäubervorrichtung (10) nach Anspruch 1, wobei das Material Platin ist.

## Revendications

1. Dispositif d'atomisation électrostatique (10) comprenant :
une électrode de décharge (20) ;
des moyens d'application de potentiel (40) configurés pour appliquer un potentiel électrique à ladite électrode de décharge (20) pour atomiser un liquide délivré à ladite électrode de décharge (20) ; et
des moyens de fourniture d'eau réduite (50) configurés pour délivrer l'eau réduite (60) en tant que dit liquide à ladite électrode de décharge (20),
dans lequel l'eau réduite (60) est définie pour contenir un matériau qui a une action de réduction et qui est dissous ou dispersé sous la forme d'une particule minuscule dans l'eau réduite (60),
**caractérisé en ce que**
ladite électrode de décharge (20) est formée de manière à avoir au moins une partie réalisée en ledit matériau,
lesdits moyens de fourniture d'eau réduite (50) sont définis par des moyens de fourniture d'eau, ladite électrode de décharge (20) et lesdits moyens d'application de potentiel (40), lesdits moyens de fourniture d'eau (50) étant configurés pour délivrer de l'eau à ladite électrode de décharge (20), et
lesdits moyens d'application de potentiel (40) étant configurés pour appliquer le potentiel électrique à ladite électrode de décharge (20) pour dissoudre ou disperser sous la forme de la particule minuscule le matériau de ladite électrode de décharge (20) dans l'eau fournie par ladite unité de fourniture d'eau et fournie à ladite électrode de décharge (20).

2. Dispositif d'atomisation électrostatique (10) selon la revendication 1, dans lequel
ledit matériau est le platine.
